# EUROPEAN PATENT APPLICATION

(11) **EP 2 980 587 A1**
(43) Date of publication of application: **03.02.2016**
(21) Application number: 14179452.9
(22) Date of filing: 01.08.2014
(51) Int. Cl.: G01N 33/564

(54) **Method to predict response to treatment with anti-TNF alpha agents**

(71) Applicant: Fundació Hospital Universitari Vall d'Hebron - Institut de Recerca, 08035 Barcelona (ES)
(72) Inventor: Marsal Barril, Sara, 08021 Barcelona (ES); Julia Cano, Antonio, 08010 Barecelona (ES)
(74) Representative: ZBM Patents - Zea, Barlocci & Markvardsen

(57) **Abstract**

There are provided *in vitro* methods for predicting response to anti-TNFα biologic treatment in a patient suffering from rheumatoid arthritis. These *in vitro* methods allow to discriminate patients that are likely to respond to monoclonal antibody-based anti-TNFα therapies from those that are not likely to respond and are therefore suitable to be treated with other anti-TNFα therapies such as Etanercept or Certolizumab-Pegol. Additionally, use of means for detecting Rheumatoid Factor and additionally anti-CCP levels in the methods of the invention are also claimed.

## Description

The present invention provides *in vitro* method to predict the response to treatment with anti-TNFα biological therapies in patients with rheumatoid arthritis. The methods have potential applications in personalized medicine.

### BACKGROUND ART

Rheumatoid arthritis (RA) is a prevalent disease in the Western world. An estimated 0.6% of the population in the USA suffers from it. It is usually treated with the Disease Modifying Anti-Rheumatic Drugs (DMARDs), which are not capable of curing the disease but which can effectively slow down its progression in a subgroup of patients. The most widely used DMARDs are methotrexate, sulfasalazine, and leflunomide.

The last years have seen the emergence of a new set of biological treatments which have proven to be very effective in the treatment of RA. Clearly, one of the most successful biological therapies in the management of RA is the Tumour Necrosis Factor alpha (TNFα) antagonists. TNFα is a proinflammatory cytokine which plays a central role in the development of many autoimmune diseasesincluding RA. Currently, the anti-TNFα biological treatments Infliximab, Adalimumab, Golimumab, Etanercept and Certolizumab-Pegol have all been approved and are being used for the management of RA. The first three are monoclonal antibodies with molecular weights over 140 KDa, while the other two are recombinant proteins of significantly smaller size (51 and 91 KDa, respectively). Etanercept is a soluble fusion protein incorporating part of the TNFα receptor, whereas Certolizumab-Pegol is a pegylated antibody fragment.

Although these biological therapies have proven to be very successful, since nearly half of all treated patients achieve an American College of Rheumatology 20% (ACR20) improvement level or even higher, they are not devoid of limitations. One of the most serious ones is unresponsiveness. A substantial proportion of treated patients show either only partial or no response to these biological therapies. Because treatment with any of these biologics is much more costly than with any DMARD, a great effort is being spent in developing methods for the prediction of responsiveness to these therapies.

Some methods for the prediction of response to anti-TNFα biologics have been disclosed in the art. Some of these methods are based on the determination of Rheumatoid Factor (RF) or anti-cyclic citrullinated peptide (anti-CCP) autoantibody levels at baseline (i.e. at the point in time when the patient has not started treatment with the anti-TNFα biologic therapy for which the response is to be estimated).

RF and anti-CCP are diagonstic markers for RA, and some studies have related their levels to response to anti-TNF treatment. However, there is a degree of controversy on how they should be used in diagnostics.

For instance, Potter M., et.al. "Association of rheumatoid factor and anti-cyclic citrullinated peptide positivity, but not carriage of shared epitope or PTPN22 susceptibility variants, with anti-tumour necrosis factor response in rheumatoid arthritis", Ann. Rheum. Dis. 2009, vol. 68, pp. 69-74, discloses that the presence of RF auto-antibodies at baseline is associated with reduced response to treatment with Infliximab, Etanercept or Adalimumab when the 28-joint Disease Activity Score (DAS28) improvement criterion is used, and the absence of RF at baseline is associated with better response to these three treatments. In this disclosure, the same behaviour was also seen for anti-CCP auto-antibodies. These results, however, were not seen when a second improvement criterion was used.

A second reference, Bobbio-Pallavicini F. et.al. "High IgA rheumatoid factor levels are associated with poor clinical response to tumour necrosis factor α inhibitors in rheumatoid arthritis" Ann. Rheum. Dis. 2007, vol. 66, pp. 302-307, discloses the association of, not the presence or absence, but levels of RF of the IgA isotype to response to treatment. It is seen that high pre-treatment levels of IgA RF are associated with a poor clinical response to the TNFα inhibitors Infliximab, Etanercept and Adalimumab. Further, it is discussed that there is a trend for the non-responders to have higher levels of both RF and anti-CCP autoantibodies, and that high-level positive patients showed a significantly lower response to the three treatments than low-level positive and negative patients.

In spite of the disclosures mentioned, other studies have drawn opposing conclusions on the role of RF and anti-CCP antibodies as predictors of response. For instance, Hyrich KL., et.al. "Predictors of response to anti-TNFα therapy among patients with rheumatoid arthritis: results from the British Society for Rheumatology Biologics Register", Rheumatology 2006, vol. 45, pp. 1558-1565, concludes that RF status at baseline is not significantly associated with response to treatment in the case of Etanercept or Infliximab, whereas Klaasen R., et.al. "The value of rheumatoid factor and anti-citrullinated protein antibodies as predictors of response to infliximab in rheumatoid arthritis: an exploratory study" Rheumatology 2011, vol. 50, pp. 1487-1493, concludes that the presence as well as the titres of RF at baseline are correlated with better response to infliximab treatment, thus giving an opposite hint to what is discussed in Potter et.al*.*(*ibid*) for this recombinant drug.

This contradictory landscape warrants the search for new and more robust methods, especially those suited to be used with all biological TNFα inhibitors, to allow a proper prediction of response to anti-TNFα therapy in RA.

### SUMMARY OF THE INVENTION

Inventors have surprisingly found for the first time, that a single threshold can be established in the titres of Rheumatoid Factor (RF) of RA patients which determines which patients are unlikely to respond to anti-TNFα therapies based on whole monoclonal antibodies (that is, Adalimumab, Infliximab and Golimumab) and remarkably are likely to respond to treatment with non-whole monoclonal antibody based anti-TNFα therapies such as Etanercept or Certolizumab-Pegol.

Thus, contrary to all of the disclosures found in the art, which group both anti-TNFα antibodies and smaller recombinant proteins such as Etanercept together in terms of response to treatment, inventors have found that there is a threshold in the titres of RF that can be used for determining which patients will not respond to treatments such as Adalimumab or Infliximab, but will instead respond to treatments such as Etanercept.

Thus, a first aspect of the invention is an *in vitro* method for predicting response to anti-TNFα biological inhibitor treatment in a patient with rheumatoid arthritis, the method comprising: a) the determination of the level of Rheumatoid Factor in a body fluid test sample of the subject; and b) the comparison of the level of Rheumatoid Factor of step (a) with a reference control level, wherein if the level determined in step (a) is higher than the reference control level, it is indicative that the patient will not respond to treatment with Adalimumab or Infliximab or Golimumab and conversely will respond to Etanercept or Certolizumab.

A second aspect of the invention is an *in vitro* method for selecting an anti-TNFα biological inhibitor treatment in a patient with rheumatoid arthritis, the method comprising: a) the determination of the level of Rheumatoid Factor in a body fluid test sample of the subject; and b) the comparison of the level of Rheumatoid Factor of step (a) with a reference control level, wherein if the level determined in step (a) is higher than the reference control level, the patient will not be recommended to be treated with Adalimumab or Infliximab or Golimumab and will be recommended to be treated with Etanercept or Certolizumab.

The application of the method of the invention can then be a useful guide in deciding before treatment begins, whether an RA patient should be given a whole antibody-based treatment or conversely should be treated with non-whole antibody therapeutic proteins such as Etanercept or Certolizumab. As it is shown below, the method is statistically robust, and therefore opens up new ways to predict response to the biological agents used in the management of RA.

A third aspect of the present invention is the use of means for detecting the level of Rheumatoid Factor and optionally the use of means for detecting the level of anti-CCP from an isolated sample in the method of the first aspect of the invention.

### BRIEF DESCRIPTION OF THE DRAWINGS

FIG.1. Graphical representation of the significance values at different anti-CCP thresholds defining high and low titer individuals. The x axis represents autoantibody titers (IU/mL) whereas the y axis represents -log10(P-value). Dotted line represents the titer value where association is maximal.
FIG.2. Scanning P-values for anti-CCP thresholds for each anti-TNF treatment separately. In all three graphs, the x axes represent autoantibody titers (IU/mL) whereas the y axes represent -log10(P-value). Dotted lines represent the titer value where association is maximal. The top graphs are for IFX (left) and ETN (right), whereas the bottom graph is for ADA.
FIG.3 Scanning P-values for RF thresholds for all patients together. The x axis represents autoantibody titers whereas the y axis represents -log10(P-value). The dotted line represents the titer value where association is maximal.
FIG.4 Scanning P-values for RF thresholds for each anti-TNF treatment separately. In all three graphs, the x axes represent autoantibody titers (IU/mL) whereas the y axes represent -log10(P-value). Dotted lines represent the titer value where association is maximal. The top graphs are for IFX (left) and ETN (right), whereas the bottom graph is for ADA.
FIG.5 Scanning P-values for RF thresholds for patients treated with monoclonal therapies only. The x axis represents autoantibody titers (IU/mL) whereas the y axis represents -log10(P-value). Dotted lines represent the titer value where association is maximal.

### DETAILED DESCRIPTION OF THE INVENTION

For the sake of understanding, the following definitions are included.

The term "Rheumatoid factor" (RF) as used herein refers to the autoantibodies that are most relevant in rheumatoid arthritis (RA). They are usually autoantibodies against the Fc (fragment crystallizable) portion of an immunoglobulin of the IgG isotype, and can be of any isotype themselves (i.e. they can be IgA, IgM, IgG, IgD or IgE). These autoantibodies are often found to be elevetad in rheumatoid arthritis patients, but can also be abnormally high in other pathologies such as viral infection, hepatitis, systemic lupus erythematosus or organ rejection. Sometimes it can even be elevated in healthy individuals. Therefore, although the presence of RF can be an indication of rheumatoid arthritis, it can be caused by other pathologies, and conversely, its absence cannot be used to rule out RA.

The term "anti-CCP" as used herein refers to a class of autoantibodies that are relevant in rheumatoid arthritis (RA). These autoantibodies are directed to self-proteins that bear citrullinated arginines. Citrullination is a post-translational modification whereby an arginine is enzymatically converted to citrulline. Citrullinated self-proteins are recognized as non-self by the immune system in RA, and thus antibodies against them are raised.

The term "anti-TNFα biological inhibitor treatment" as used herein indicates a treatment based on the administration of any of the biological anti-TNFα drugs currently available in the market, that is: the monoclonal antibodies Infliximab (trade name Remicade), Adalimumab (trade name Humira) and Golimumab (trade name Simponi), the monoclonal antibody fragment Certolizumab-Pegol (trade name Cimzia) and the fusion protein Etanercept (trade name Enbrel). More specifically the term or expression "therapy with whole monoclonal antibodies" relates to the administration of anti-TNFα biologic substances (TNFα antagonists) which are monoclonal antibodies commonly used in the treatment of RA. On the other hand, the expression "therapy with non-whole monoclonal antibodies" relates to the administration of anti-TNFα biologic substances (TNFα antagonists) which are fragments of monoclonal antibodies or fusion proteins commonly used in the treatment of RA, that is, Certolizumab-Pegol and Etanercept.

The term "predicting response to anti-TNFα biological inhibitor treatment" as used herein refers to the determination of the likelihood that the patient will respond either favourably or unfavourably to the administered anti-TNFα biological inhibitor treatment. The response to these treatments can be measured by applying the European League Against Rheumatism (EULAR) response criteria. The EULAR response criteria classifies the patient individuals as non-, moderate or good responders depending on the change and the level of the Disease Activity Score (DAS).

The term "reference control level" referred to in the methods of the invention is to be understood as a predefined value of a given molecular marker, Rheumatoid Factor in the present case, which is derived from the levels of said molecular marker in a sample or group of samples. The samples are taken from a subject or group of subjects wherein the presence, absence, stage, or course of the disease has properly been determined previously. The subject or subjects from whom the "reference control level" is derived may include subject/s wherein the condition is absent, subject/s wherein the condition is present, or both. The person skilled in the art, making use of the general knowledge, is able to choose the subject or group of subjects more adequate for obtaining the control levels for each of the methods of the present invention. Methods for obtaining the reference value from the group of subjects selected are well-known in the state of the art (Burtis C. A. et al., 2008, Chapter 14, section "Statistical Treatment of Reference Values"). In a particular case the "reference control level" for soluble Rheumatoid Factor is a cut-off value defined by means of a conventional ROC analysis (Receiver Operating Characteristic analysis). As the skilled person will appreciate, optimal cut-off values will be defined according to the particular application of the method: target population for the prediction, balance between specificity and sensitivity, and others.

The term "body fluid test sample" is to be understood as a liquid originating from inside the living body. It includes fluids that are excreted or secreted from the body as well as body water that normally is not.

The term "antibody or a fragment thereof which specifically binds to Rheumatoid Factor" is to be understood as any immunoglobulin or fragment thereof able to selectively bind the RF. It includes monoclonal and polyclonal antibodies. The term "fragment thereof" encompasses any part of an antibody having the size and conformation suitable to bind an epitope of RF. Suitable fragments include F(ab), F(ab'), nanobodies and Fv. An "epitope" is the part of the antigen being recognized by the immune system (B-cells, T-cells or antibodies).

The term "antibody or a fragment thereof which specifically binds to anti-CCP" is to be understood as any immunoglobulin or fragment thereof able to selectively bind anti-CCP antibodies. It includes monoclonal and polyclonal antibodies. The term "fragment thereof" encompasses any part of an antibody having the size and conformation suitable to bind an epitope of anti-CCP. Suitable fragments include F(ab), F(ab'), nanobodies and Fv. An "epitope" is the part of the antigen being recognized by the immune system (B-cells, T-cells or antibodies).

As mentioned above, a first aspect of the invention is an *in vitro* method for predicting response to anti-TNFα biological inhibitor treatment in a patient with rheumatoid arthritis, the method comprising: a) the determination of the level of Rheumatoid Factor in a body fluid test sample of the subject; and b) the comparison of the level of Rheumatoid Factor of step (a) with a reference control level, wherein if the level determined in step (a) is higher than the reference control level, it is indicative that the patient will not respond to treatment with Adalimumab or Infliximab or Golimumab and conversely will respond to Etanercept or Certolizumab.

In a particular embodiment of the first aspect of the invention, when using the experimental detection techniques and materials disclosed in the present application (examples section), the reference control level (cutoff value) for RF would be 148IU/ml. This means that patients with values above 148IU/ml will probably not respond to treatment with Adalimumab, Infliximab or Golimumab, and conversely will probably respond to treatment with Etanercept or Certolizumab.

In a particular embodiment of the first aspect, the invention provides an *in vitro* method for predicting response to anti-TNFα biological inhibitor treatment in a patient with rheumatoid arthritis according to the first aspect, comprising: a) the determination of the level of Rheumatoid Factor in a body fluid test sample of the subject; b) the comparison of the level of step (a) with a reference control level, wherein if the level determined in step (a) is higher than the reference control level, it is indicative that the patient will not respond to treatment with Adalimumab or Infliximab or Golimumab and conversely will respond to Etanercept or Certolizumab, and wherein if the level determined in step (a) is lower than the reference control level, then it is determined the level of anti-CCP in a body fluid test sample of the subject; and c) comparing the level of step (b) for anti-CCP with its reference control level, wherein if the level determined in step (b) is higher than its reference control level, it is indicative that the patient will respond to treatment with Adalimumab or Infliximab or Golimumab and will not respond to Etanercept or Certolizumab and wherein if the level determined in step (b) is lower than its reference control level, it is indicative that the patient will not respond to Adalimumab or Infliximab or Golimumab and will respond to Etanercept or Certolizumab.

In a particular embodiment of the first aspect of the invention, when using the experimental detection techniques and materials disclosed in the present application (examples section), the reference control level (cutoff value) for RF would be 148IU/ml and the reference control level (cutoff value) for a-CCP would be 730IU/ml. This means that patients with values above 148IU/ml will probably not respond to treatment with Adalimumab, Infliximab or Golimumab, and conversely will probably respond to treatment with Etanercept or Certolizumab. And for patients with RF values below 148IU/ml, the level of anti-CCP is determined, wherein patients whose anti-CCP level is higher than 730IU/ml will probably respond to treatment with Adalimumab or Infliximab or Golimumab and will not respond to Etanercept or Certolizumab, and patients whose anti-CCP level is lower than 730IU/ml will probably not respond to treatment with Adalimumab or Infliximab or Golimumab and will probably respond to Etanercept or Certolizumab

Thus, in a particular embodiment of the first aspect of the invention, if the level determined of Rheumatoid Factor in step (a) is lower than the reference control level, then it is further determined the level of anti-CCP in a body fluid test sample of the subject.

As also mentioned above, a second aspect of the present invention is an *in vitro* method for selecting an anti-TNFα biological inhibitor treatment in a patient with rheumatoid arthritis, the method comprising: a) the determination of the level of Rheumatoid Factor in a body fluid test sample of the subject; and b) the comparison of the level of Rheumatoid Factor of step (a) with a reference control level, wherein if the level determined in step (a) is higher than the reference control level, the patient will not be recommended to be treated with Adalimumab or Infliximab or Golimumab and will be recommended to be treated with Etanercept or Certolizumab.

In a particular embodiment of the second aspect, the invention provides an in vitro method for selecting an anti-TNFα biological inhibitor treatment in a patient with rheumatoid arthritis , the method comprising:
a) the determination of the level of Rheumatoid Factor in a body fluid test sample of the subject; b) the comparison of the level of step (a) with a reference control level, wherein if the level determined in step (a) is higher than the reference control level, the patient will not be recommended to be treated with Adalimumab or Infliximab or Golimumab and will be recommended to be treated with Etanercept or Certolizumab, and wherein if the level determined in step (a) is lower than the reference control level, then it is determined the level of anti Cyclic Citrullinated Peptide (anti-CCP) in a body fluid test sample of the subject; and
c) the comparison of the level of step (b) for anti-CCP with its reference control level, wherein if the level determined in step (b) is higher than its reference control level, then the patient will be recommended to be treated with Adalimumab or Infliximab or Golimumab and will not be recommended to be treated with Etanercept or Certolizumab and wherein if the level determined in step (b) is lower than its reference control level, the patient will not be recommended to be treated with Adalimumab or Infliximab or Golimumab and will be recommended to be treated with Etanercept or Certolizumab.

In a particular embodiment of the second aspect of the invention, when using the experimental detection techniques and materials disclosed in the present application (examples section), the reference control level (cutoff value) for RF would be 148IU/ml and the reference control level (cutoff value) for anti-CCP would be 730IU/ml.

In a particular embodiment of the first and second aspects of the invention, the sample is selected from the group consisting of plasma, blood serum, whole blood, saliva, and urine.

In another particular embodiment of the first and second aspects, the step of determining the level of Rheumatoid Factor comprises the use of an anti-Rheumatoid Factor antibody or a fragment thereof which specifically binds to said Rheumatoid Factor.

In another particular embodiment of the first and second aspects, the step of determining the level of Rheumatoid Factor comprises the use of an anti-Rheumatoid Factor antibody or a fragment thereof which specifically binds to said Rheumatoid Factor, and the step of determining the level of anti-CCP comprises the use of an anti anti-CCP antibody or a fragment thereof which specifically binds to said anti-CCP.

In another particular embodiment of the first and second aspects, the step of determining the level of RF comprises the use of any of the platforms available for RF detection such as those from Cobas-Roche, Euro-Diagnostica, Phadia, Abbott Diagnostics, ImmunoLab and Abnova, and the step of determining the level of anti-CCP comprises the use of any of the platmfors available for anti-CCP detection such as those from INOVA Diagnostics, Euro-Diagnostica, Axis-Shield, Phadia, Cobas-Roche and Abbot Diagnostics.

The antibody or fragment thereof for detecting Rheumatoid Factor and optionally antibody or fragment thereof for detecting anti-CCP can be included in a kit. The kit may additionally comprise means (additives, solvents) to visualize the antigen-antibody interactions (dipsticks, chemiluminescent reagents, turbidimetric reagents, etc.).

In yet another particular embodiment, the determination of the Rheumatoid Factor levels and optionally the determination of anti-CCP levels in any of the methods provided is made following an immunochemistry technique.

Immunochemistry refers to the process of detecting antigens (e.g., proteins) in a sample by exploiting the principle of antibodies binding specifically to antigens. Visualising an antibody-antigen interaction can be accomplished in a number of ways. In the most common instance, an antibody is conjugated to an enzyme, such as peroxidase, that can catalyse a colour-producing reaction. Alternatively, the antibody can also be tagged to a fluorophore, such as fluorescein or rhodamine. The immunohistochemistry technique can be direct or indirect. The direct method is a one-step staining method and involves a labeled antibody (e.g. FITC-conjugated antiserum) reacting directly with the antigen. While this technique utilizes only one antibody and therefore is simple and rapid, the sensitivity is lower due to little signal amplification, such as with indirect methods, and is less commonly used than indirect methods. The indirect method involves an unlabeled primary antibody (first layer) that binds to the target antigen in the sample and a labeled secondary antibody (second layer) that reacts with the primary antibody. This method is more sensitive than direct detection strategies because of signal amplification due to the binding of several secondary antibodies to each primary antibody if the secondary antibody is conjugated to the fluorescent or enzyme reporter. Further amplification can be achieved if the secondary antibody is conjugated to several biotin molecules, which can recruit complexes of avidin-, streptavidin or Neutravidin-enzyme. The indirect method, aside from its greater sensitivity, also has the advantage that only a relatively small number of standard conjugated (labeled) secondary antibodies needs to be generated. With the direct method, it would be necessary to label each primary antibody for every antigen of interest.

The antibodies used for specific detection can be polyclonal or monoclonal. Polyclonal antibodies are made by injecting animals with peptide antigen and, after a secondary immune response is stimulated, isolating antibodies from whole serum. Thus, polyclonal antibodies are a heterogeneous mix of antibodies that recognize several epitopes. Monoclonal antibodies show specificity for a single epitope and are therefore considered more specific to the target antigen than polyclonal antibodies.

As mentioned above, a third aspect of the present invention is the use of means for detecting the level of Rheumatoid Factor and optionally the use of means for detecting the level of anti-CCP from an isolated sample in the method of the first aspect of the invention.

In a particular embodiment of the second aspect, the means comprise an antibody or a fragment thereof able to bind to Rheumatoid Factor and optionally an antibody or a fragment thereof able to bind to anti-CCP.

In another particular embodiment of the second aspect, the means to carry out the invention form part of a kit.

Throughout the description and claims the word "comprise" and variations of the word, are not intended to exclude other technical features, additives, components, or steps. Furthermore, the word "comprise" and its variations encompasses the term "consisting of". Additional objects, advantages and features of the invention will become apparent to those skilled in the art upon examination of the description or may be learned by practice of the invention. The following examples are provided by way of illustration, and they are not intended to be limiting of the present invention. Furthermore, the present invention covers all possible combinations of particular and preferred embodiments described herein.

### EXAMPLES

### A) Patients and Methods

All Rheumatoid Arthritis patients (RA) were diagnosed according to the American College of Rheumatology diagnostic criteria, with >2years of disease evolution, Caucasian, with Spanish ancestry (up to 2 generations) and were >18 years old.

Patients were treated with one of the following anti-TNF therapies: Adalimumab (Humira, Abbot, ADA for short), Infliximab (Remicade, Merck, IFX for short) and Etanercept (Enbrel, Pfizer, ETN for short).

A total of 289 RA patients from which anti-TNF treatment response at week 12 was available was used for the present analysis. The cohort included a total of 80.2% women (close to expected 3:1 ratio of disease prevalence).

Whole blood samples were obtained for each patient using Vacutainer extraction tubes (Becton Dickinson) with EDTA anticoagulant agent. After 24 hrs at room temperature, tubes were centrifuged and the supernatant (i.e. plasma) separated and stored at -80°C until use. The plasma samples from all patients were obtained between 2007 and 2010.

All plasma samples were simultaneously analyzed for anti-CCP titers and RF titers. For anti-CCP quantiation the Cobas anti-CCP (Roche, REF: 05031656) *in vitro* kit was used. For the RF quantiation the Cobas Rheumatoid Factors (Roche, REF: 20764574) *in vitro* kit was used. The raw data was obtained at the Vall d'Hebron Hospital and submitted to the GRR-VHIR for analysis.

Using the systems described above, titers of anti-Cyclic Citrullinated Peptide (anti-CCP) and Rheumatoid Factor (RF) antibodies were determined for all plasma samples. Accordingly, autoantibody analysis yielded:
- Anti-CCP positive: 79.6%
- RF positive: 76.8%
- Anti-CCP positive OR RF positive: 88.6%
- Anti-CCP positive AND RF positive: 67.8%

Treatment response was defined according to the EULAR response criteria. According to disease activity at weeks 0 (baseline) and 12 of anti-TNF treatment, patients are categorized as GOOD, MODERATE and NONE EULAR responders. In the present study we have:
- EULAR GOOD RESPONDERS: 32.5% (n=94)
- EULAR MODERATE RESPONDERS: 40.1% (n=116)
- EULAR NONE RESPONDERS: 27.3% (n=79)

Disease activity is measured using the DAS28 score (Disease Activity Score defined by EULAR). Both basal DAS28 as well as DAS28 (i.e. Basal DAS28 - week12 DAS28), are normally distributed variables, according to Shapiro-Wilk's test.

The study was performed according to the response to the first anti-TNF reatment. The percentage of the three main anti-TNF treatments is:
- Adalimumab (ADA): 26.2% (n=74)
- Etanercept (ETN): 34.7% (n=98).
- Infliximab (IFX): 39% (n=110).

For the present study, patients were categorized into a binary response, that is, GOOD and MODERATE EULAR were aggregated into a RESPONDER group (72.7% of patients), while NONE was used as the NON RESPONDER group (27.3% of patients).

| Response | ADA | ETN | IFX |
|---|---|---|---|
| NON-RESP | 21.3 | 23.5 | 33.6 |
| RESP | 78.7 | 76.5 | 66.4 |

The percentage of NON-RESPONDERS in the IFX group is higher (P<0.05) than the other 2 treatment gruops. Since it was the first biological therapy to be approved, patients having a more longstanding disease might have been included -hence less responsive-, reducing the apparent efectiveness of the therapy.

All statistical analyses were performed using the R statistical language system (v.2.10.1). Significance between group categorizations was determined using Fisher's exact test. The search for new optimal autoantibody thresholds was performed by testing for association with treatment response (Fisher's test) over different titer (U/mL) thresholds. Significance values were recorded and plotted to identify the most discriminating threshold. According to this optimal threshold patients were reclassified as being HI (high titer) or LO (low titer).

### B) Identification of biomarker profile

B.1. Association of standard autoantibody measures.

It was studied the association of anti-TNF response with the level of each antibody (i.e. RF and anti-CCP titers). This type of study is a quantitative trait analysis and was performed using linear regression.
- RF and GLOBAL: There is no association of all anti-TNFs with response (P=0.21)
- RF and ADA: Non significative (P=0.12)
- RF and ETN: Non significative (P=0.22)
- RF and IFX: There is a significant association with non-response to therapy (P=0.0033)
- CCP and GLOBAL: Non significative (P=0.63)
- CCP and ETN: Non significative (P=0.66)
- CCP and IFX: Non significative (P=0.74)
- CCP and ADA: Non significative (P=0.20)

We find that higher levels of RF are associated with non-response in IFX-treated patients. ETN is no longer signicative if we use the antibody levels. It is only associated when RF is dichotomized into positive and negative groups.

### B.2. Identification of new biomarker profile

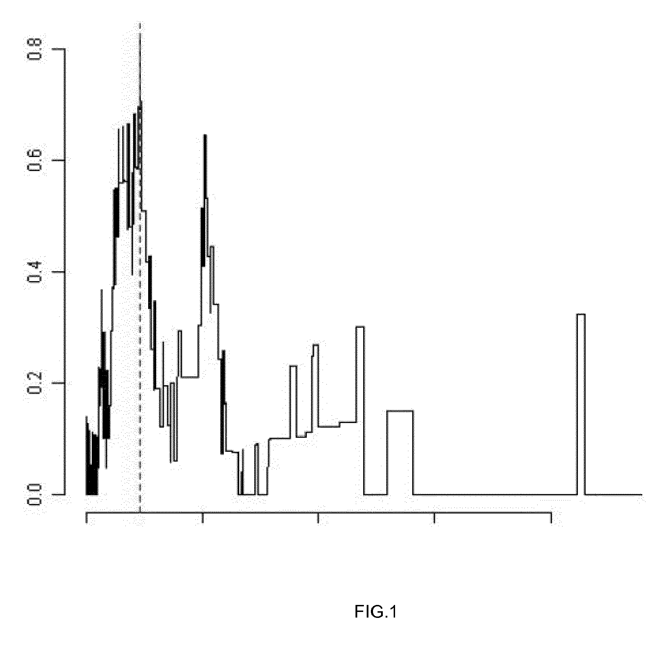

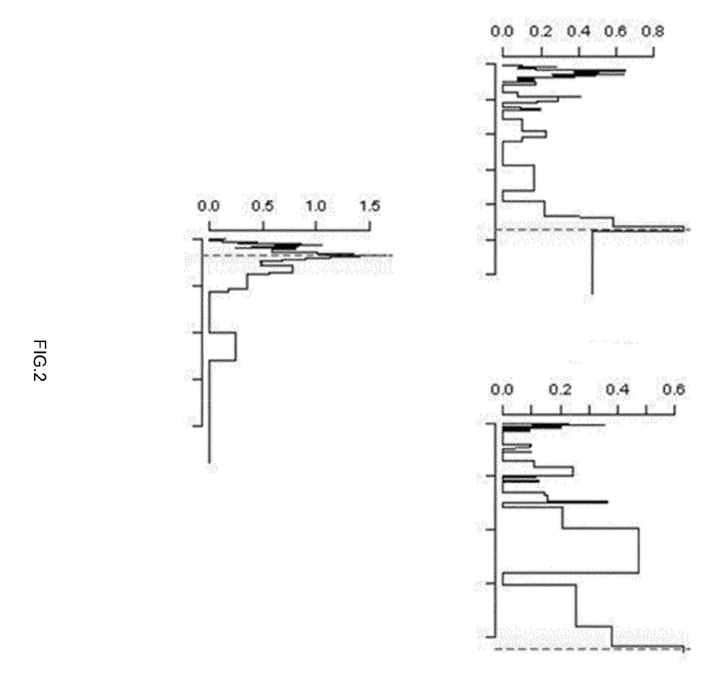

Using automated search methods our objective was to identify new profiles that can best discriminate treatment response. First, we screened anti-CCP. As can be seen in FIG.1, no significant antibody threshold was found taking into account all treatments together. However, when performing the automated search method for each treatment separatel, we saw a clear different behaviour.. As can be seen in FIG.2, we found that there is a specific treshold in anti-CCP levels that is associated to response in ADA-treated patients (P=0.022). 92% of patients with high levels of anti-CCP responded to therapy compared to 70% of patients with low levels of anti-CCP. Low levels also included CCP- patients. Importantly, using the standard negative-positive classication, there was clearly no signicance for anti-CCP biomarker and ADA treatment (P=0.56).

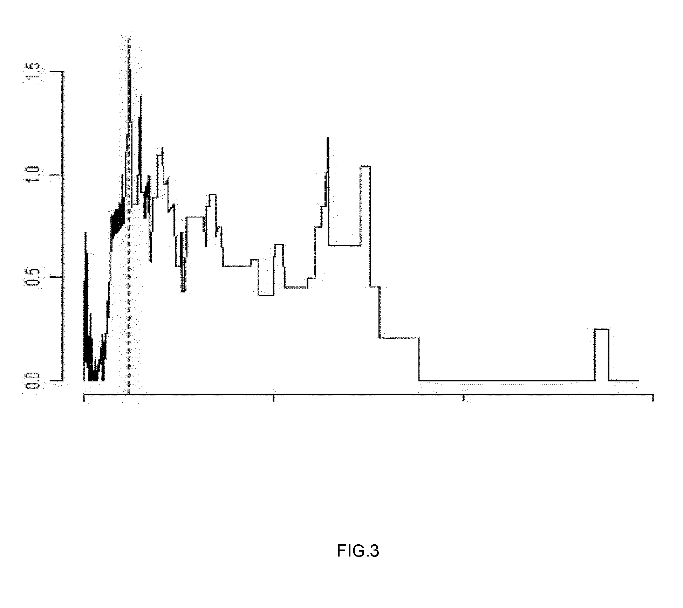

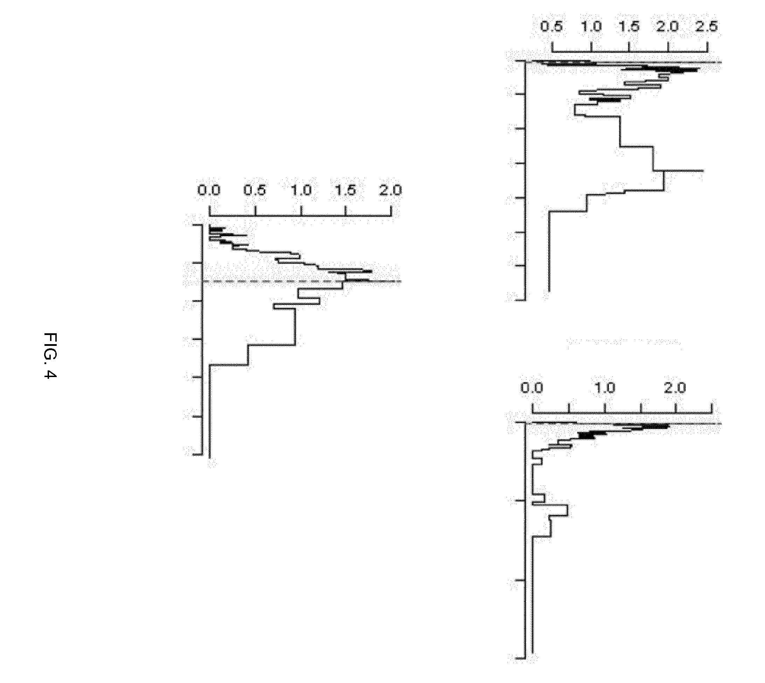

When applying the same screen for RF, we also found new and significant results. As can be seen in FIG. 3, in this case, there was an autoantibody threshold at which patients were significantly discriminated by response (P=0.02). We also investigated the association within each anti-TNF treatment. As seen in FIG. 4, in this subtype analysis we found more significant results. For IFX, the association was highly significant (P=0.002), with 40% of RA patients with high levels of RF not responding to treatment, compared to 10% patients in the low level group.

For ETN the association was also highly significant (P=0.002) but, importantly, in the inverse sense compared to IFX. Only 15% of RA patients with high RF levels did not respond to ETN, compared to 50% patients with low levels.

ADA had a similar behaviour to IFX. It was also highly associated (P=0.009), with 62% of patients with high levels of RF not responding to treatment, compared to 16% of patients with low levels.

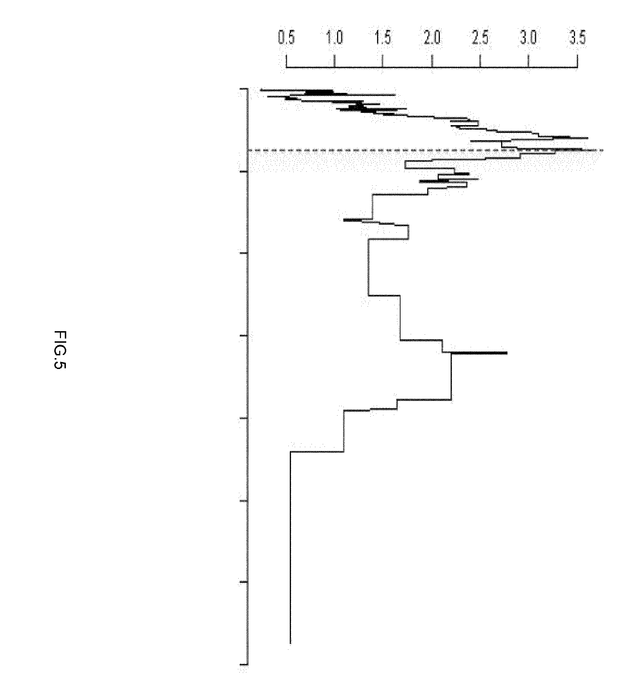

Given that both IFX and ADA showed a similar relation to RF levels, we evaluated the combination of both monoclonal-type treatments. As seen in FIG. 5, the association with this combined group showed the highest significance of all analyses (P=0.00023). In this combination, 61 % of RF-High patients did not respond to therapy, compared to only 23% of patients. Therefore, patients having RF-High values would be the optimal patients to begin non-monoclonal therapies like Etanercept or Certolizumab.

The results point to RF as the main discriminating variable. Patients who have high RF values (14% of all) will clearly be prone to have a bad response to the two monoclonal antibodies (ADA and IFX) compared to patients who have low RF values (86%, P= 0.00023).

### REFERENCES CITED IN THE APPLICATION

Potter M., et.al. "Association of rheumatoid factor and anti-cyclic citrullinated peptide positivity, but not carriage of shared epitope or PTPN22 susceptibility variants, with anti-tumour necrosis factor response in rheumatoid arthritis", Ann. Rheum. Dis. 2009, vol. 68, pp. 69-74
Bobbio-Pallavicini F., et.al. "High IgA rheumatoid factor levels are associated with poor clinical response to tumour necrosis factor α inhibitors in rheumatoid arthritis" Ann. Rheum. Dis. 2007, vol. 66, pp. 302-307
Hyrich KL., et.al. "Predictors of response to anti-TNFα therapy among patients with rheumatoid arthritis: results from the British Society for Rheumatology Biologics Register", Rheumatology 2006, vol. 45, pp. 1558-1565
Klaasen R., et.al. "The value of rheumatoid factor and anti-citrullinated protein antibodies as predictors of response to infliximab in rheumatoid arthritis: an exploratory study" Rheumatology 2011, vol. 50, pp. 1487-1493
Burtis C. A. et al., 2008, Chapter 14, section "Statistical Treatment of Reference Values").

## Claims

1. An *in vitro* method for predicting response to anti-TNFα biological inhibitor treatment in a patient with rheumatoid arthritis, the method comprising:
a) the determination of the level of Rheumatoid Factor in a body fluid test sample of the subject; and
b) the comparison of the level of Rheumatoid Factor of step (a) with a reference control level, wherein if the level determined in step (a) is higher than the reference control level, it is indicative that the patient will not respond to treatment with Adalimumab or Infliximab or Golimumab and conversely will respond to Etanercept or Certolizumab.

2. The in vitro method for predicting response to anti-TNFα biological inhibitor treatment in a patient with rheumatoid arthritis according to claim 1, comprising:
a) the determination of the level of Rheumatoid Factor in a body fluid test sample of the subject;
b) the comparison of the level of step (a) with a reference control level, wherein if the level determined in step (a) is higher than the reference control level, it is indicative that the patient will not respond to treatment with Adalimumab or Infliximab or Golimumab and conversely will respond to Etanercept or Certolizumab, and wherein if the level determined in step (a) is lower than the reference control level, then it is determined the level of anti Cyclic Citrullinated Peptide (anti-CCP) in a body fluid test sample of the subject; and
c) the comparison of the level of step (b) for anti-CCP with its reference control level, wherein if the level determined in step (b) is higher than its reference control level, it is indicative that the patient will respond to treatment with Adalimumab or Infliximab or Golimumab and will not respond to Etanercept or Certolizumab and wherein if the level determined in step (b) is lower than its reference control level, it is indicative that the patient will not respond to Adalimumab or Infliximab or Golimumab and will respond to Etanercept or Certolizumab.

3. An *in vitro* method for selecting an anti-TNFα biological inhibitor treatment in a patient with rheumatoid arthritis, the method comprising:
a) the determination of the level of Rheumatoid Factor in a body fluid test sample of the subject; and
b) the comparison of the level of Rheumatoid Factor of step (a) with a reference control level, wherein if the level determined in step (a) is higher than the reference control level, the patient will not be recommended to be treated with Adalimumab or Infliximab or Golimumab and will be recommended to be treated with Etanercept or Certolizumab.

4. The in vitro method for selecting an anti-TNFα biological inhibitor treatment in a patient with rheumatoid arthritis according to claim 3, the method comprising:
a) the determination of the level of Rheumatoid Factor in a body fluid test sample of the subject;
b) the comparison of the level of step (a) with a reference control level, wherein if the level determined in step (a) is higher than the reference control level, the patient will not be recommended to be treated with Adalimumab or Infliximab or Golimumab and will be recommended to be treated with Etanercept or Certolizumab, and wherein if the level determined in step (a) is lower than the reference control level, then it is determined the level of anti Cyclic Citrullinated Peptide (anti-CCP) in a body fluid test sample of the subject; and
c) the comparison of the level of step (b) for anti-CCP with its reference control level, wherein if the level determined in step (b) is higher than its reference control level, then the patient will be recommended to be treated with Adalimumab or Infliximab or Golimumab and will not be recommended to be treated with Etanercept or Certolizumab and wherein if the level determined in step (b) is lower than its reference control level, the patient will not be recommended to be treated with Adalimumab or Infliximab or Golimumab and will be recommended to be treated with Etanercept or Certolizumab.

5. The *in vitro* method according to claims 1-4, wherein the sample is selected from the group consisting of plasma, blood serum and whole blood.

6. The in vitro method according to any one of claims 1-5, wherein the step of determining the level of Rheumatoid Factor comprises the use of an anti-Rheumatoid Factor antibody or a fragment thereof which specifically binds to said Rheumatoid Factor, and wherein the step of determining the level of anti-CCP comprises the use of an anti anti-CCP antibody or a fragment thereof which specifically binds to said anti-CCP.

7. The in vitro method according to claim 6, wherein the determination comprises an immunochemistry technique.

8. Use of means for detecting the level of Rheumatoid Factor and optionally use of means for detecting the level of anti-CCP from an isolated sample in the in vitro method of any one of the claims 1-7.

9. The use according to claim 8, wherein said means comprise an antibody or a fragment thereof able to bind to Rheumatoid Factor and optionally an antibody or a fragment thereof able to bind to anti-CCP.

10. The use according to any one of claims 8-9, wherein said means form part of a kit.
